# EUROPEAN PATENT SPECIFICATION

(11) **EP 0 341 773 B1**
(45) Date of publication and mention of the grant of the patent: **11.09.1996**
(21) Application number: 89201122.2
(22) Date of filing: 28.04.1989
(51) Int. Cl.: C07D 307/32, C07D 309/30

(54) **Process for the preparation of lactones**
Verfahren zur Herstellung von Lactonen
Procédé pour la préparation de lactones

(30) Priority: 10.05.1988 GB 8811023; 10.05.1988 GB 8811024
(43) Date of publication of application: 15.11.1989
(73) Proprietor: SHELL INTERNATIONALE RESEARCH MAATSCHAPPIJ B.V., NL-2596 HR Den Haag (NL)
(72) Inventor: Drent, Eit, NL-1031 CM Amsterdam (NL)

(56) References cited:
- EP-A- 105 704
- EP-A- 0 106 379
- EP-A- 0 176 370
- US-A- 3 859 319
- J. CHEM. SOC., CHEM. COMMUN., no. 8, 15th April 1985, pages 511-512, London, GB; H. ALPER et al.: "Palladium-catalysed conversion of alkenols into five- and six-membered ring lactones at room temperature and atmospheric pressure"
- F. Falbe: "New Syntheses with Carbon Monoxide", Springer Verlag (1980),pp. 280-284;ISBN 0-387-09674-4
- Houben-Weyl:"Sauerstoffverbindungen I, part 2, Georg Thieme Verlag Stuttgart (1963), p.569

## Description

The present invention relates to the production of optionally substituted lactones having 4 or 5 carbon atoms in the ring by the reaction of optionally substituted higher alkenols, in which the hydroxy group is more than 3 carbon atoms remote from the double bond, with carbon monoxide.

From US Patent No. 4,634,780, it is known that lactones having a 5 or 6 membered ring can be prepared by reacting, inter alia, an alkenol containing 5 or more aliphatic carbon atoms with carbon monoxide. However the following restriction is made with respect to the alkenols which may be used in the process: the olefinic unsaturation should not be located more than three carbon atoms from the hydroxy substituent. Said patent specification does not teach in any way the conversion of alkenols having 5 or more aliphatic carbon atoms in which the aliphatic unsaturation is more than 3 carbon atoms removed from the hydroxy substituent. Moreover the process described in said patent specification should be carried out in the presence of oxygen in order to obtain acceptable, yet moderate, product yields. The catalytic system used in this reaction consists of
1. a protonic acid,
2. one or more metals selected from the group consisting of palladium, rhodium, ruthenium, iridium cobalt or compounds thereof, and
3. one or more metals selected from the group consisting of copper, molybdenum and iron.

It will be appreciated that the before-mentioned process has several unfavourable characteristics i.a. as starting compounds only alkenols can be used which have the olefinic unsaturation no more than three carbon atoms removed from the hydroxy substituent and a complex catalyst system is used.

Consequently, due to an increasing demand for lactone products which can be used as solvents and useful intermediates for chemical synthesis, there is a need for a more universal and economical production process.

It now has been surprisingly found that optionally substituted lactones having four or five carbon atoms in the ring can be produced with improved selectivity and improved yields by reaction of optionally substituted alkenols, in which the hydroxy group is more than 3 carbon atoms remote from the double bond, in the presence of a relatively simple catalytic system which is believed to cause isomerization in situ by shifting the carbon to carbon double bond of an alkenol, in which the olefinic unsaturation is more than three carbon atoms removed from the hydroxy substituent, to an alkenol in which the olefinic unsaturation is three carbon atoms removed from the hydroxy substituent.

More particularly the invention relates to a process for the preparation of lactones having four or five carbon atoms in the ring or a mixture thereof, which process comprises reacting an alkenol, having the general formula: 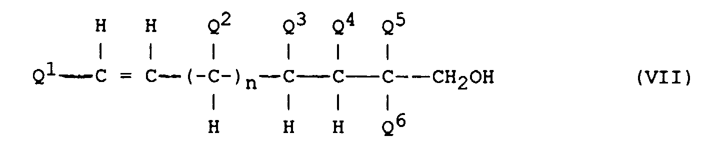 in which n is 0 or an integer from 1 to 25; Q¹ represents a H atom or a C₁₋₃₀ alkyl group; Q², Q³, Q⁴, Q⁵ and Q⁶ each independently represent a H atom or a C₁₋₄ alkyl group; with the proviso that Q³ and Q⁴ do not represent a lower alkyl group simultaneously, with a carbon monoxide containing gas, whereby a catalytic system is used obtainable by combining:
(a) a palladium compound
(b) a bidentate phosphine, arsine or stibine, and
(c) a protonic acid having a pKa less than 2.

It has been surprisingly found that the process according to the present invention allows the preparation of lactones having 4 or 5 carbon atoms in the ring with an extremely high selectivity.

The selectivity to a desired compound can be expressed as 100% x p:q, in which "p" is the amount of starting compound that has been converted into that desired compound and "q" is the total amount of starting compound that has been converted.

It will be appreciated that the process of the present invention is very attractive because it is carried out in the presence of a relatively simple catalyst system. Moreover, this process shows an extremely high selectivity to lactones having four or five carbon atoms in the ring thus minimizing the formation of byproducts, whereas it enables the conversion of higher alkenols, unlike the process of US-4,634,780.

The process of the present invention is suitably carried out without the presence of oxygen thus minimizing the danger of explosion and the aselective oxidation of carbon monoxide to carbon dioxide.

The lactones produced according to the present invention are interesting solvents and very useful intermediates. For example lactones can be converted to enol ethers, hydroxylesters and lactams.

The process according to the present invention is carried out in the liquid phase in which the palladium compound may be heterogeneous but is preferably homogeneous. Suitable homogeneous palladium compounds are the palladium(II) salts of for instance, nitric acid, sulphuric acid, or preferably of alkanoic acids having no more than 12 carbon atoms per molecule. A preferably used palladium compound is palladium(II) acetate.

Other examples of suitable palladium(II) compounds are palladium(II) formate and palladium(II) propanoate. Salts of hydrohalogenic acids may also be used in principle but are not preferred due to their corrosive properties.

Moreover, palladium complexes may be used, for instance palladium acetylacetonate, tetrakistriphenylphosphine palladium acetate or bis-triphenylphosphine-palladium sulphate. A mixture of palladium compounds may be used as component (a).

As component (b) a bidentate chelating ligand, a monodentate chelating ligand, or a combination of a bidentate chelating ligand and a monodentate chelating ligand may be used. If the double bond is more than four carbon atoms remote from the hydroxy group in the alkenol, the use of bidentate ligands is preferred because of their higher double bond shifting activity.

The bidentate chelating ligand comprises an organic compound containing as coordinating atoms at least two atoms of phosphorus, arsenic, or antimony which are connected through a divalent organic bridging group having at least two carbon atoms in the bridge.

The two or more coordinating atoms may be the same or different, for example two phosphorus atoms, an arsenic and a phosphorus atom or an arsenic and an antimony atom, and are preferably both phosphorus.

The bidentate chelating ligand preferably has the general formula I: 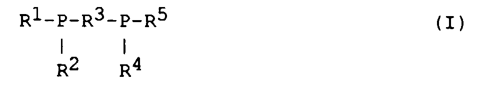 in which R¹, R², R⁴ and R⁵ represent identical or different optionally substituted hydrocarbon groups and the bridging group R³ represents a chain consisting of two to six optionally substituted carbon atoms.

Any substituents present in the bidentate chelating ligand preferably do not cause steric hindrance to the formation of complex compounds with the palladium(II) compound.

Hydrocarbyl groups R¹, R², R⁴ and R⁵ will as a rule contain 2 to 18 carbon atoms, preferably 6 to 14 carbon atoms. Aryl groups are the most suitable, in particular the phenyl group. Preferred bridging groups -R³- are those having the formula --(CR⁶R⁷-)-ₙ in which R⁶ and R⁷ are hydrogen atoms or optionally substituted hydrocarbyl groups preferably offering no steric hindrance and n is an integer of at least two, preferably not more than 5, and most preferably 2, 3 or 4. Substituents R⁶ and R⁷ are preferably hydrogen atoms. The bridging groups R³ may also form part of a cyclic structure, e.g. an aromatic or cycloaliphatic group. The carbon to carbon bond or bonds in the bridge may be saturated or unsaturated and in the bridge, or in the cyclic or non-cyclic groups attached to the bridge, one or more hetero atoms, e.g. sulphur, oxygen, iron or nitrogen atoms, may replace carbon atoms, provided that both phosphorus atoms are linked to a carbon atom present in the bridge. Examples of suitable chelating ligands are
1,3-di(diphenylphosphino)propane,
1,4-di(diphenylphosphino)butane,
2,3-dimethyl-1,4-di(diphenylphosphino)butane,
1,5-di(methylphenylphosphino)pentane,
1,4-di(dicyclohexylphosphino)butane,
1,5-di(dinaphthylphosphino)pentane,
1,3-di(di-p-tolylphosphino)propane,
1,4-di(di-p-methoxyphenylphosphino)butane,
1,2-di(diphenylphosphino)ethene,
2,3-di(diphenylphosphino)-2-butene,
1,3-di(diphenylphosphino)-2-oxopropane,
2-methyl-2-(methyldiphenylphosphino)-1,3-di(diphenylphosphino)-propane (acting like a bidentate ligand),
o,o'-di(diphenylphosphino)biphenyl,
1,2-di(diphenylphosphino)benzene,
2,3-di(diphenylphosphino)naphthalene,
1,2-di(diphenylphosphino)cyclohexane,
2,2-dimethyl-4,5-di(diphenylphosphino)dioxolane and
bis(3-(diphenylphosphino)phenyl)iron(II).

Very good results have been obtained with 1,4-di(diphenylphosphino)butane or 1,3-di(diphenylphosphino)propane. A mixture of chelating ligands of the general formula I may be used.

The monodentate phosphine, when present, has the general formula II: 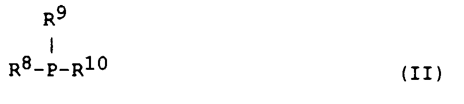 in which R⁸, R⁹, and R¹⁰ each individually represent an optionally substituted aryl group.

The substituted or unsubstituted aryl groups R⁸, R⁹ and R¹⁰ of the phosphine of the general formula II each preferably contain not more than 18, in particular in the range of from 6 to 14 carbon atoms. Examples of suitable R⁸, R⁹ and R¹⁰ groups are the naphthyl group and in particular, the phenyl group. Suitable substituents are halogen atoms and alkyl, aryl, alkoxy, carboxy, carbalkoxy, acyl, trihalogenmethyl, cyano, dialkylamino, sulphonylalkyl and alkanoyloxy groups.

Examples of suitable phosphines are tri-p-tolylphosphine, tris(p-chlorophenyl)phosphine, tris-p-methoxyphenylphosphine, o-diphenylphosphinobenzoic acid and preferably triphenylphosphine. A mixture of phosphines of the general formula II may be used.

The protonic acid having a pKa of less than 2 preferably has an anion which is non-coordinating, by which is meant that little or no covalent interaction takes place between the palladium and the anion of the protonic acid. Thus, hydrohalogenic acids are less preferred as component (c).

A preferred group of acids has the general formula III 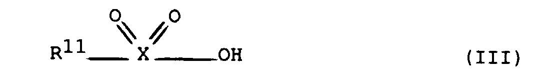 in which X represents a sulphur or a chlorine atom and, if X represents a chlorine atom, R¹¹ represents an oxygen atom and, if X represents a sulphur atom, R¹¹ represents an OH group or an optionally substituted hydrocarbyl group.

When the latter acids are used in the process according to the invention, their anions can be considered to be non-coordinating.

In the acids having the general formula III, the optionally substituted hydrocarbyl group represented by R¹¹ is preferably an alkyl, aryl, aralkyl or alkaryl group having 1-30, in particular 1-14, carbon atoms. The hydrocarbyl group may, for example, be substituted with halogen atoms, in particular fluorine atoms. Preferred acids of the general formula III are perchloric acid, sulphuric acid, p-toluenesulphonic acid and trifluoromethanesulphonic acid. p-Toluenesulphonic acid is particularly preferred. Another suitable acid is 2-hydroxypropane-2-sulphonic acid. The acid of the general formula III can also be an ion exchanger containing sulphonic acid groups, such as Amberlite 252 H ("Amberlite" is a trade mark). In that case, the hydrocarbyl group R¹¹ is a polymeric hydrocarbyl group, for example a polystyrene group substituted with sulphonic acid groups. Further examples of suitable acids are those that can be formed, possibly in situ, by interacting a Lewis acid such as BF₃, AsF₅, SbF₅, PF₅, TaF₅ or NbF₅ with a Broensted acid such as a hydrohalogenic acid, in particular HF, fluorosulphonic acid, phosphoric acid or sulphuric acid. Specific examples of acids of the latter type are H₂SiF₆, HBF₄, HPF₆ and HSbF₆. Examples of suitable sulphonic acids are fluorosulphonic acid and chloro-sulphonic acid. Other examples of suitable acids are trichloroacetic acid, trifluoroacetic acid, dichloroacetic acid and difluoroacetic acid. A mixture of protonic acids having a pKₐ of less than 2 may be used as component (c).

As will be appreciated, the process according to the present invention may be carried out using a variety of optionally substituted alkenols in which the hydroxy group is more than 3 carbon atoms remote from the double bond. The process can be schematically represented by means of equation A:

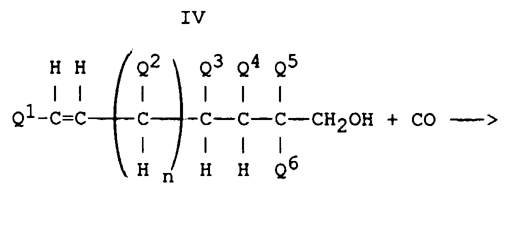

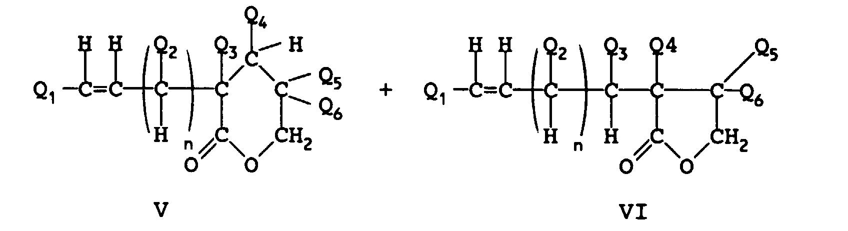

With the term lower alkyl as used throughout the specification is meant an alkyl containing 1-4 carbon atoms. Among these alkyl groups methyl groups and ethyl groups are preferred in particular methyl groups. However, Q², Q³, Q⁴, Q⁵ and, Q⁶ most preferably represent hydrogen atoms.

It will be appreciated too that the carbon monoxide molecule involved in the reaction according to equation A will only bind to a secondary carbon atom involved in the carbon to carbon double bond. Therefore the product of the general formula V can only be formed if Q³ represents hydrogen and the product of the general formula VI can only be formed if Q⁴ represents hydrogen. Consequently a mixture of the products V and VI is formed if Q³ and Q⁴ both represent hydrogen.

Alkenols which are useful reactants are for example:
5-hexen-1-ol,
6-hepten-1-ol, 5-hepten-1-ol, 7-octen-1-ol,
6-octen-1-ol, 5-octen-1-ol, 8-nonen-1-ol,
7-nonen-1-ol, 6-nonen-1-ol, 5-nonen-1-ol, 9-decen-1-ol,
8-decen-1-ol, 7-decen-1-ol, 6-decen-1-ol, 5-decen-1-ol,
10-undecen-1-ol, 9-undecen-1-ol, 8-undecen-1-ol,
9-undecen-1-ol, 11-dodecen-1-ol, 10 dodencen-1-ol,
9 dodecen-1-ol, 8-dodencen-1-ol, 7-dodecen-1-ol,
6-dodecen-1-ol, 5-dodencen-1-ol, 13-tetradecen-1-ol,
12-tetradecen-1-ol, 11-tetradecen-1-ol, 10-tetradecen-1-ol,
9-tetradecen-1-ol, 8-tetradecen-1-ol, 7-tetradecen-1-ol,
6-tetradecen-1-ol, 5-tetradecen-1-ol, 14-pentadecen-1-ol,
15-hexadecen-1-ol, 16-heptadecen-1-ol, 17-octadecen-1-ol,
9-octadecen-1-ol, 18-nonadecen-1-ol, 19-eicosen-1-ol, and 20-heneicosen-1-ol.

In a preferred embodiment, the invention concerns a process whereby a catalytic system is used obtainable by combining:
(a) a palladium compound, in a molar ratio to the alkenol of from 10⁻¹ to 10⁻⁶,
(b) a bidentate phosphine, arsine or stibine, in a molar ratio to component (a) of from 1 to 200, and
(c) a protonic acid having a pKa less than 2, in a molar ratio to component (b) of more than 1.

The process according to the invention is suitably carried out in the presence of a solvent, which is preferably aprotic. Examples of such solvents are hydrocarbons, such as hexane, heptane, octane, benzene, toluene, the three xylenes, ethylbenzene, cumene, cyclohexane and decalin; halogenated hydrocarbons, such as dichloromethane, chloroform, 1,2-dichloroethane, perfluoroalkanes, chlorobenzene and the three dichlorobenzenes; sulphones such as diethyl sulphone, diisopropyl sulphone and tetrahydrothiophene 1,1-dioxide (also referred to as "sulfolane"); N,N-dialkyl-substituted amides such as N,N-dimethylformamide and N-methylpyrrolidone; esters such as methyl benzoate, ethyl acetate and amyl acetate; ethers such as diethyl ether, 3,6-dioxaoctane, methyl tert.-butylether, tetrahydrofuran, diisopropyl ether, 1,4-dioxane, 2,5,8-trioxanonane (also referred to as "diglyme"), diphenyl ether and anisole. Very good results have been obtained with ethers.

In the process according to the invention as carbon monoxide containing gas may be used carbon monoxide in pure form or diluted with an inert gas, such as nitrogen, noble gases or carbon dioxide or hydrogen, e.g. in the form of synthesis gas.

Generally the presence of more than 20% hydrogen is undesirable, since under the reaction conditions it may cause hydrogenation of the olefinic compound. Generally the preference is given to carbon monoxide or a carbon monoxide containing gas which contains less than 5% hydrogen.

The process according to the present invention can be carried out at a temperature and a pressure which are not critical and may vary within wide ranges. The temperature may suitably vary in the range of from 20 °C to 250 °C and preferably from 50 °C to 200 °C. The pressure may suitably vary in the range of from 1 to 100 bar and preferably from 10 to 75 bar.

Reaction time may vary over a wide range from about 15 minutes to 8 hours.

The process according to the present invention may be carried out batchwise, semi-continuously or continuously. When operating batchwise, the catalytic system, the starting alkenol and the solvent are charged to a reactor to form a liquid phase therein, the reactor is pressurized with carbon monoxide and heated to the desired temperature. When operating continuously, the liquid components can be charged to the reactor continuously to form a liquid phase therein and the carbon monoxide continuously introduced into the reactor to contact the liquid phase containing the catalyst. The gaseous reactants can be withdrawn from the reactor as a separate effluent, cooled, depressurized and the carbon monoxide can be recycled for further contacting.

The lactones may be isolated from the reaction mixture in any suitable manner, for example by means of extraction or by distillation, obtaining a distillate fraction containing the lactones and a bottom fraction containing the catalytic system. Suitably, a solvent is chosen that substantially remains in the bottom fraction. Preferably, at least a portion of the bottom fraction containing solvent and catalytic system is re-used in the process according to the invention.

The following Examples further illustrate the invention.

### EXAMPLE 1

A 300 ml magnetically stirred Hastelloy autoclave ("Hastelloy" is a trade mark) was charged with the following materials:
- anisole 40 ml
- 10-undecen-1-ol 10 ml
- Pd(Ac)₂ 0.4 mmol
- P(Ph)₂-(CH₂)₄-P(Ph)₂ 1.6 mmol
- para-toluenesulphonic acid 4 mmol.

The autoclave was flushed with carbon monoxide, pressurized with carbon monoxide until a partial pressure of 40 bar was obtained, heated to a temperature of 140 °C and kept at this temperature for 5 hours.

The autoclave was cooled to an ambient temperature and vented and the content was analyzed by gas liquid chromatography. At the end of the reaction, the conversion of 10-undecen-1-ol was 65% with a selectivity to 3-octyl-2-tetrahydrofuranone of 68% and to 3-heptyl-2-tetrahydropyranone of 19%.

### EXAMPLE 2

The experiment was carried out as described in example 1 with the difference that 2 mmol PPh₃ were also added to the reaction mixture.

At the end of the reaction, the conversion of 10-undecen-1-ol was 44% with a selectivity to 3-octyl-2-tetrahydrofuranone of 69% and to 3-heptyl-2-tetrahydropyranone of 23%.

### EXAMPLE 3

The experiment was carried out as described in example 1 with cis-9-octadecen-1-ol (oleyl alcohol) as the alkenol.

At the end of the reaction, the conversion of cis-9-octadecen-1-ol was 86% with a selectivity of to 3-pentadecyl-2-tetrahydrofuranone of 66% and to 3-tetradecyl-2-tetrahydropyranone of 25%.

## Claims

1. Process for the preparation of lactones having four or five carbon atoms in the ring or a mixture thereof, which process comprises reacting an optionally substituted alkenol, having the general formula: 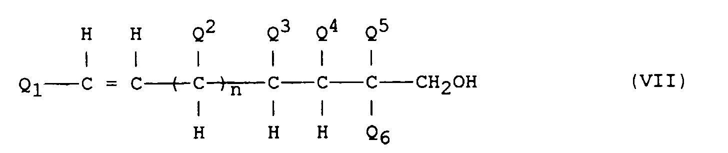 in which n is 0 or an integer from 1 to 25; Q¹ represents a H atom or a C₁₋₃₀ alkyl group; Q², Q³, Q⁴, Q⁵ and Q⁶ each independently represent a H atom or a C₁₋₄ alkyl group; with the proviso that Q³ and Q⁴ do not represent a lower alkyl group simultaneously with a carbon monoxide containing gas whereby a catalytic system is used obtainable by combining:
(a) a palladium compound
(b) a phosphine, arsine or stibine, and
(c) a protonic acid having a pKa less than 2.

2. A process as claimed in claim 1, characterized in that the palladium compound is a palladium(II)salt of an alkanoic acid having no more than 12 carbon atoms per molecule.

3. A process as claimed in claim 1 or 2, characterized in that component (b) is a chelating ligand comprising an organic compound containing as coordinating atoms at least two atoms selected from the group consisting of phosphorus, arsenic or antimony which are connected through a divalent organic bridging group having at least two carbon atoms in the bridge.

4. A process as claimed in claim 3, characterized in that the chelating ligand has the general formula I 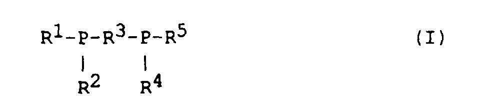 in which R¹, R², R⁴ and R⁵ represent identical or different optionally substituted hydrocarbyl groups and R³ represents a chain consisting of two to six optionally substituted carbon atoms.

5. A process as claimed in claim 4, characterized in that the chelating ligand is 1,4-di(diphenylphosphino)butane or 1,3-di(diphenylphosphino)propane.

6. A process as claimed in any of claims 1-5, characterized in that component (c) is a protonic acid having a non-coordinating anion.

7. A process as claimed in claim 6, characterized in that component (c) has the general formula III 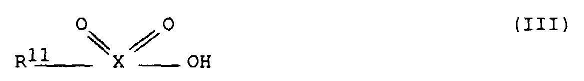 in which X represents a sulphur or a chlorine atom and, if X represents a chlorine atom, R¹¹ represents an oxygen atom and, if X represents a sulphur atom, R¹¹ represents an OH group or an optionally substituted hydrocarbyl group.

8. A process as claimed in claim 7, characterized in that component (c) is p-toluenesulphonic acid.

9. A process as claimed in any of claims 1-8, characterized in that a catalytic system is used obtainable by combining:
(a) a palladium compound, in a molar ratio to the alkenol of from 10⁻¹ to 10⁻⁶,
(b) a bidentate phosphine, arsine or stibine, in a molar ratio to component (a) of from 1 to 200, and
(c) a protonic acid having a pKa less than 2, in a molar ratio to component (b) of more than 1.

10. A process as claimed in any of claims 1-9, characterized in that the catalyst system comprises a monodentate chelating ligand having the general formula II 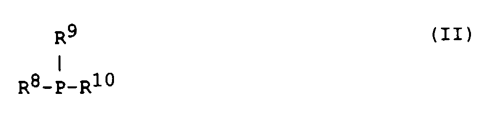 in which R⁸, R⁹, and R¹⁰ each individually represent an optionally substituted aryl group.

11. A process as claimed in claim 10, characterized in that the aryl groups of the monodentate ligand compound are phenyl groups.

12. A process as claimed in any of claims 1-11, characterized in that it is carried out at a temperature in the range of 20 to 250 °C and at a pressure in the range from 1 to 100 bar.

## Patentansprüche

1. Verfahren zur Herstellung von Lactonen mit vier oder fünf Kohlenstoffatomen im Ring oder einer Mischung daraus, bei dem man ein gegebenenfalls substituiertes Alkenol mit der allgemeinen Formel: 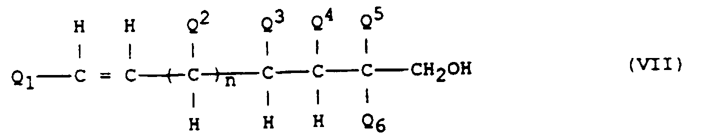 worin n für 0 oder eine ganze Zahl von 1 bis 25 steht; Q¹ ein H-Atom oder eine C₁₋₃₀-Alkylgruppe bedeutet; Q², Q³, Q⁴, Q⁵ und Q⁶ jeweils unabhängig voneinander ein H-Atom oder eine C₁₋₄-Alkylgruppe bedeuten; mit der Maßgabe, daß Q³ und Q⁴ nicht gleichzeitig eine niedere Alkylgruppe bedeuten, mit einem kohlenmonoxidhaltigen Gas umsetzt, wobei man ein katalytisches System verwendet, das erhältlich ist durch Kombinieren von:
(a) einer Palladiumverbindung,
(b) einem Phosphin, Arsin oder Stibin, und
(c) einer Protonensäure mit einem pKa-Wert unter 2.

2. Verfahren nach Anspruch 1, dadurch gekennzeichnet, daß man als Palladiumverbindung ein Palladium(II)-Salz einer Alkansäure mit bis zu 12 Kohlenstoffatomen pro Molekül einsetzt.

3. Verfahren nach Anspruch 1 oder 2, dadurch gekennzeichnet, daß man als Komponente (b) einen Chelatliganden, bei dem es sich um eine organische Verbindung handelt, die als koordinierende Atome mindestens zwei Atome ausgewählt aus der Gruppe, bestehend aus Phosphor, Arsen oder Antimon, enthält, die über eine zweiwertige organische Brückengruppe mit mindestens zwei Kohlenstoffatomen in der Brücke verbunden sind, einsetzt.

4. Verfahren nach Anspruch 3, dadurch gekennzeichnet, daß man einen Chelatliganden der allgemeinen Formel I 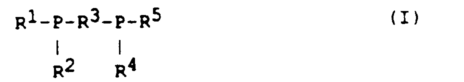 worin R¹, R², R⁴ und R⁵ gleiche oder verschiedene, gegebenenfalls substituierte Kohlenwasserstoffgruppen bedeuten und R³ eine aus zwei bis sechs gegebenenfalls substituierten Kohlenstoffatomen bestehende Kette bedeutet, einsetzt.

5. Verfahren nach Anspruch 4, dadurch gekennzeichnet, daß man als Chelatliganden 1,4-Di(diphenylphosphino)butan oder 1,3-Di(diphenylphosphino)propan einsetzt.

6. Verfahren nach einem der Ansprüche 1-5, dadurch gekennzeichnet, daß man als Komponente (c) eine Protonensäure mit einem nicht koordinierenden Anion einsetzt.

7. Verfahren nach Anspruch 6, dadurch gekennzeichnet, daß man eine Komponente (c) der allgemeinen Formel III 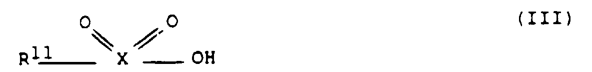 worin X ein Schwefel- oder ein Chloratom bedeutet und in dem Fall, daß X ein Chloratom bedeutet, R¹¹ ein Sauerstoffatom bedeutet, und in dem Fall, daß X ein Schwefelatom bedeutet, R¹¹ eine OH-Gruppe oder eine gegebenenfalls substituierte Kohlenwasserstoffgruppe bedeutet, einsetzt.

8. Verfahren nach Anspruch 7, dadurch gekennzeichnet, daß man als Komponente (c) p-Toluolsulfonsäure einsetzt.

9. Verfahren nach einem der Ansprüche 1-8, dadurch gekennzeichnet, daß man ein katalytisches System verwendet, das erhältlich ist durch Kombinieren von:
(a) einer Palladiumverbindung in einem Molverhältnis zum Alkenol von 10⁻¹ bis 10⁻⁶,
(b) einem zweizähnigen Phosphin, Arsin oder Stibin in einem Molverhältnis zur Komponente (a) von 1 bis 200, und
(c) einer Protonensäure mit einem pKa-Wert unter 2 in einem Molverhältnis zur Komponente (b) von über 1.

10. Verfahren nach einem der Ansprüche 1-9, dadurch gekennzeichnet, daß das Katalysatorsystem einen einzähnigen Chelatliganden der allgemeinen Formel II 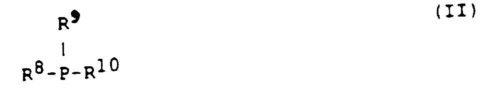 worin R⁸, R⁹ und R¹⁰ jeweils für sich eine gegebenenfalls substituierte Arylgruppe bedeuten, enthält.

11. Verfahren nach Anspruch 10, dadurch gekennzeichnet, daß es sich bei den Arylgruppen der einzähnigen Ligandenverbindung um Phenylgruppen handelt.

12. Verfahren nach einem der Ansprüche 1-11, dadurch gekennzeichnet, daß man es bei einer Temperatur im Bereich von 20 bis 250 °C und bei einem Druck im Bereich von 1 bis 100 bar durchführt.

## Revendications

1. Procédé de préparation de lactones possédant 4 ou 5 atomes de carbone dans le cycle ou un mélange de celles-ci, caractérisé en ce que l'on fait réagir un alcénol répondant à la formule générale suivante : 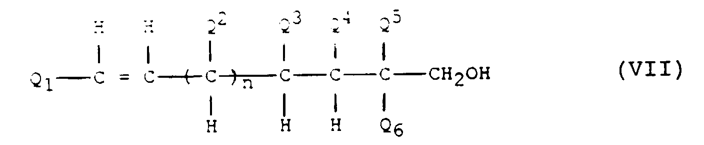 dans laquelle n est égal à 0 ou représente un nombre entier dont la valeur varie de 1 à 25; Q¹ représente un atome d'hydrogène ou un radical alkyle en C₁ à C₃₀; Q², Q³, Q⁴, Q⁵ et Q⁶ représentent, chacun indépendamment, un atome d'hydrogène ou un radical alkyle en C₁ à C₄; avec la condition que Q³ et Q⁴ ne représentent pas simultanément chacun un radical alkyle inférieur, avec un gaz contenant du monoxyde de carbone, réaction en vue de laquelle on utilise un système catalytique que l'on peut obtenir en combinant :
(a) un composé du palladium,
(b) une stibine, une arsine, ou une phosphine, bidentate, et
(c) un acide protonique possédant un pKa inférieur à 2.

2. Procédé suivant la revendication 1, caractérisé en ce que le composé de palladium est un sel de palladium(II) d'un acide alcanoïque ne comportant pas plus de 12 atomes de carbone par molécule.

3. Procédé suivant la revendication 1 ou 2, caractérisé en ce que le composant (b) est un ligand chélateur constitué d'un composé organique contenant, à titre d'atomes de coordinence, au moins 2 atomes choisis dans le groupe formé par le phosphore, l'arsenic et l'antimoine, qui sont reliés par l'intermédiaire d'un groupe de pontage organique bivalent possédant au moins 2 atomes de carbone dans le pont.

4. Procédé suivant la revendication 3, caractérisé en ce que le ligand chélateur répond à la formule générale I : 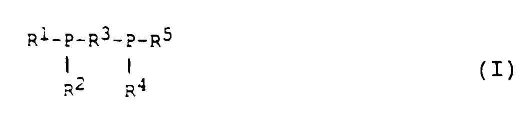 dans laquelle R¹, R², R⁴ et R⁵ représentent des radicaux hydrocarbonés éventuellement substitués, identiques ou différents et le groupe de pontage R³ représente une chaîne constituée de 2 à 6 atomes de carbone éventuellement substitués.

5. Procédé suivant la revendication 4, caractérisé en ce que le ligand chélateur est le 1,4-di(diphénylphosphino)butane ou le 1,3-di(diphénylphosphino)-propane.

6. Procédé suivant l'une quelconque des revendications 1 à 5, caractérisé en ce que le composé (c) est un acide protonique possédant un anion non coordinent.

7. Procédé suivant la revendication 6, caractérisé en ce que le composant (c) répond à la formule générale III : 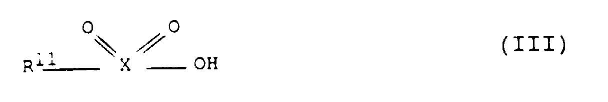 dans laquelle X représente un atome de soufre ou un atome de chlore et, lorsque X représente un atome de chlore, R¹¹ représente un atome d'oxygène et, lorsque X représente un atome de soufre, R¹¹ représente un radical OH ou un radical hydrocarbyle éventuellement substitué.

8. Procédé suivant la revendication 7, caractérisé en ce que le composant (c) est l'acide p-toluène-sulfonique.

9. Procédé suivant l'une quelconque des revendications 1 à 8, caractérisé en ce que l'on utilise un système catalytique que l'on peut obtenir en combinant :
(a) un composé du palladium, dans un rapport molaire à l'alcénol de 10⁻¹ à 10⁻⁶,
(b) une stibine, une arsine, ou une phosphine, bidentate, dans un rapport molaire au composant (a) de 1 à 200, et
(c) un acide protonique possédant un pKa inférieur à 2, dans un rapport molaire au composant (b) supérieur à 1.

10. Procédé suivant l'une quelconque des revendications 1 à 9, caractérisé en ce que le système catalytique comprend un ligand chélateur monodentate répondant à la formule générale II : 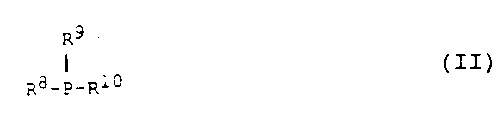 dans laquelle R⁸, R⁹ et R¹⁰ représentent chacun individuellement un radical aryle éventuellement substitué.

11. Procédé suivant la revendication 10, caractérisé en ce que les radicaux aryle du composé du type ligand monodentate sont des radicaux phényle.

12. Procédé suivant l'une quelconque des revendications 1 à 11, caractérisé en ce qu'on l'entreprend à une température qui varie de 20 à 250°C et sous une pression qui fluctue de 1 à 100 bars.
